# EUROPEAN PATENT APPLICATION

(11) **EP 4 011 912 A1**
(43) Date of publication of application: **15.06.2022**
(21) Application number: 21743662.5
(22) Date of filing: 22.01.2021
(51) Int. Cl.: C07K 16/18, C12N 5/10, C12N 1/15, C12N 1/19, C12N 1/21, C12N 15/13, C12N 15/63, G01N 33/53, G01N 33/531, G01N 33/543

(54) **METHOD AND KIT FOR DETECTING HUMAN ALPHA-DEFENSIN HD5, AND ANTIBODIES USED IN SAID METHOD AND KIT**

(30) Priority: 23.01.2020 JP 2020009553
(71) Applicant: National University Corporation Hokkaido University, Sapporo-shi, Hokkaido 060-0808 (JP)
(72) Inventor: AYABE Tokiyoshi, Sapporo-shi, Hokkaido 060-0808 (JP); NAKAMURA Kiminori, Sapporo-shi, Hokkaido 060-0808 (JP)
(74) Representative: Prüfer & Partner mbB Patentanwälte · Rechtsanwälte
(86) International application number: PCT/JP2021/002250
(87) International publication number: WO 2021/149798

(57) **Abstract**

[Problem] The purpose of the present invention is to provide a method for detecting, in a selective and simple manner, an oxidized form of HD5 which is human α-defensin, that is, HD5 having intramolecular disulfide bonds that function beneficially in the human body.

[Solution] The present invention relates to: two kinds of novel monoclonal antibodies that contain amino acid sequences of SEQ ID NO: 1-6 or 7-12 as a CDR and that specifically bind to human α-defensin HD5 having intramolecular disulfide bonds; an immunological detection method using the antibodies; and a kit containing the antibodies.

SEQUENCE LISTING

<110>  National University Corporation Hokkaido University

<120>  Method and kit for detecting human alpha defensin HD5, and
       antibody used therein

<130>  P19HU07WO

<160>  19

<170>  PatentIn version 3.5

<210>  1
<211>  5
<212>  PRT
<213>  Mus musculus

<400>  1

Ser Tyr Gly Ile Ser
1               5

<210>  2
<211>  17
<212>  PRT
<213>  Mus musculus

<400>  2

Glu Ile Tyr Pro Arg Ser Gly Asn Thr Tyr Tyr Asn Glu Lys Phe Lys
1               5                   10                  15

Gly

<210>  3
<211>  10
<212>  PRT
<213>  Mus musculus

<400>  3

Asp Tyr Asp Gly Phe Pro Tyr Phe Asp Tyr
1               5                   10

<210>  4
<211>  15
<212>  PRT
<213>  Mus musculus

<400>  4

Arg Ala Ser Glu Ser Val Asp Ser Tyr Gly Tyr Ser Phe Met His
1               5                   10                  15

<210>  5
<211>  7

<212>  PRT
<213>  Mus musculus

<400>  5

Arg Ala Ser Asn Leu Glu Ser
1               5


<210>  6
<211>  9
<212>  PRT
<213>  Mus musculus

<400>  6

Gln Gln Ser Asn Glu Asp Pro Arg Thr
1               5


<210>  7
<211>  5
<212>  PRT
<213>  Mus musculus

<400>  7

Gly Ser Tyr Met Asn
1               5


<210>  8
<211>  17
<212>  PRT
<213>  Mus musculus

<400>  8

Glu Ile Asn Pro Ser Thr Gly Gly Thr Thr His Asn Gln Lys Phe Lys
1               5                   10                  15


Ala




<210>  9
<211>  12
<212>  PRT
<213>  Mus musculus

<400>  9

Asp Tyr Ala Gly Ser Tyr Asp Trp Tyr Phe Asp Val
1               5                   10


<210>  10
<211>  11
<212>  PRT
<213>  Mus musculus

<400>  10

Lys Ala Ser Gln Asp Ile Asn Ser Tyr Leu Ser
1               5                   10

<210>  11
<211>  7
<212>  PRT
<213>  Mus musculus

<400>  11

Arg Ala Asn Arg Leu Val Asp
1               5

<210>  12
<211>  9
<212>  PRT
<213>  Mus musculus

<400>  12

Leu Gln Tyr Asp Glu Phe Pro Tyr Thr
1               5

<210>  13
<211>  119
<212>  PRT
<213>  Mus musculus

<400>  13

Gln Val Gln Leu Gln Gln Ser Gly Ala Glu Leu Ala Arg Pro Gly Ala
1               5                   10                  15

Ser Val Lys Leu Ser Cys Lys Ala Ser Gly Tyr Thr Phe Thr Ser Tyr
            20                  25                  30

Gly Ile Ser Trp Val Lys Gln Arg Thr Gly Gln Gly Leu Glu Trp Ile
        35                  40                  45

Gly Glu Ile Tyr Pro Arg Ser Gly Asn Thr Tyr Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Asp Tyr Asp Gly Phe Pro Tyr Phe Asp Tyr Trp Gly Gln Gly
            100                 105                 110

Thr Thr Leu Thr Val Ser Ser

        115

<210>  14
<211>  111
<212>  PRT
<213>  Mus musculus

<400>  14

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
            20                  25                  30

Gly Tyr Ser Phe Met His Trp Tyr Gln Gln Lys Pro Gly Gln Pro Pro
        35                  40                  45

Lys Leu Leu Ile Tyr Arg Ala Ser Asn Leu Glu Ser Gly Ile Pro Ala
    50                  55                  60

Arg Phe Ser Gly Ser Gly Ser Arg Thr Asp Phe Thr Leu Thr Ile Asn
65                  70                  75                  80

Pro Val Glu Ala Asp Asp Val Ala Thr Tyr Tyr Cys Gln Gln Ser Asn
                85                  90                  95

Glu Asp Pro Arg Thr Phe Gly Gly Gly Thr Lys Leu Glu Ile Lys
            100                 105                 110

<210>  15
<211>  121
<212>  PRT
<213>  Mus musculus

<400>  15

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Ser Phe Thr Gly Ser
            20                  25                  30

Tyr Met Asn Trp Val Lys Gln Ser Pro Glu Lys Ser Leu Glu Trp Ile
        35                  40                  45

Gly Glu Ile Asn Pro Ser Thr Gly Gly Thr Thr His Asn Gln Lys Phe
    50                  55                  60

Lys Ala Lys Ala Thr Leu Thr Val Asp Thr Ser Ser Ser Thr Phe Tyr
65                  70                  75                  80

Met Gln Leu Lys Ser Leu Thr Ser Glu Asp Ser Ala Val Tyr Tyr Cys
                85                  90                  95

Ala Arg Asp Tyr Ala Gly Ser Tyr Asp Trp Tyr Phe Asp Val Trp Gly
            100                 105                 110

Ala Gly Thr Thr Val Thr Val Ser Ser
        115                 120

<210> 16
<211> 107
<212> PRT
<213> Mus musculus

<400> 16

Asp Ile Lys Met Thr Gln Ser Pro Ser Ser Met Tyr Ala Ser Leu Gly
1               5                   10                  15

Glu Arg Val Thr Ile Thr Cys Lys Ala Ser Gln Asp Ile Asn Ser Tyr
            20                  25                  30

Leu Ser Trp Phe Gln Gln Lys Pro Gly Lys Ser Pro Lys Thr Leu Ile
        35                  40                  45

Tyr Arg Ala Asn Arg Leu Val Asp Gly Val Pro Ser Arg Phe Ser Gly
    50                  55                  60

Ser Gly Ser Gly Gln Asp Tyr Ser Leu Thr Ile Ser Ser Leu Glu Tyr
65                  70                  75                  80

Glu Asp Met Gly Ile Tyr Tyr Cys Leu Gln Tyr Asp Glu Phe Pro Tyr
                85                  90                  95

Thr Phe Gly Gly Gly Thr Lys Leu Glu Ile Ile
            100                 105

<210> 17
<211> 94
<212> PRT
<213> Homo sapiens

<400> 17

Met Arg Thr Ile Ala Ile Leu Ala Ala Ile Leu Leu Val Ala Leu Gln
1               5                   10                  15

Ala Gln Ala Glu Ser Leu Gln Glu Arg Ala Asp Glu Ala Thr Thr Gln
            20                  25                  30

Lys Gln Ser Gly Glu Asp Asn Gln Asp Leu Ala Ile Ser Phe Ala Gly
        35                  40                  45

Asn Gly Leu Ser Ala Leu Arg Thr Ser Gly Ser Gln Ala Arg Ala Thr
    50                  55                  60

Cys Tyr Cys Arg Thr Gly Arg Cys Ala Thr Arg Glu Ser Leu Ser Gly
65                  70                  75                  80

Val Cys Glu Ile Ser Gly Arg Leu Tyr Arg Leu Cys Cys Arg
                85                  90

<210>  18
<211>  32
<212>  PRT
<213>  Homo sapiens

<400>  18

Ala Thr Cys Tyr Cys Arg Thr Gly Arg Cys Ala Thr Arg Glu Ser Leu
1               5                   10                  15

Ser Gly Val Cys Glu Ile Ser Gly Arg Leu Tyr Arg Leu Cys Cys Arg
            20                  25                  30

<210>  19
<211>  32
<212>  PRT
<213>  Homo sapiens

<400>  19

Ala Phe Thr Cys His Cys Arg Arg Ser Cys Tyr Ser Thr Glu Tyr Ser
1               5                   10                  15

Tyr Gly Thr Cys Thr Val Met Gly Ile Asn His Arg Phe Cys Cys Leu
            20                  25                  30

## Description

### FIELD

The present invention relates to a method and a kit for detecting an oxidized form of human α-defensin HD5, and an antibody used in the method and the kit.

### BACKGROUND

The relationship between an intestinal environment and various diseases including lifestyle-related diseases is known in recent years. α-defensin has attracted attention as a host-derived substance capable of influencing on an intestinal environment, in particular intestinal microbiota.

α-defensin is an antimicrobial peptide exhibiting strong bactericidal activity against pathogenic bacteria, and is produced by Paneth cells, which are a lineage of cells constituting the mucosal epithelium of the small intestine. In recent years, cryptdin 4, which is mouse α-defensin, has been found to exhibit selective bactericidal activity depending on its higher order structure. Cryptdin 4 with a normal higher order structure, that is, an oxidized form of cryptdin 4 with three intramolecular disulfide bonds, exhibits poor bactericidal activity against many commensal bacteria including Bifidobacterium bifidum and Bacteroides fragilis, while a reduced form of cryptdin 4 without any intramolecular disulfide bond exhibits stronger bactericidal activity against commensal bacteria than the oxidized form. Both of the oxidized and reduced forms of cryptdin 4 exhibit strong bactericidal activity against pathogenic bacteria such as Salmonella enterica serovar Typhimurium and Staphylococcus aureus (Non Patent Literature 1). The foregoing indicates that maintaining the high order structure of α-defensin, that is, having three intramolecular disulfide bonds is important to exhibit its selective bactericidal activity, and that α-defensin with a normal high order structure can control intestinal microbiota appropriately and contribute to the maintenance of the intestinal environment favorable for the host.

Therefore, measurement of the oxidized form of α-defensin with a normal high order structure is considered to enable exploration of substances effective to control the intestinal environment as well as investigation of new methods for preventing diseases relevant to the intestinal environment and strategies to cure such diseases. Until now, the inventors of the present invention have reported a method for selectively detecting an oxidized form of mouse α-defensin (Non Patent Literature 2), but a method for selectively detecting the oxidized form of human α-defensin remains unknown.

### CITATION LIST

### NON PATENT LITERATURE

Non Patent Literature 1: K. Masuda et al., J. Innate Immun. 2011, 3, 315-326.
Non Patent Literature 2: K. Nakamura et al., Analytical biochemistry. 2013, 443(2), 124-131.

### SUMMARY

### TECHNICAL PROBLEM

An object of the present invention is to provide a method for detecting selectively and simply an oxidized form of human α-defensin HD5, that is, HD5 with intramolecular disulfide bonds useful to function in human bodies.

### SOLUTION TO PROBLEM

The inventors of the present invention produced monoclonal antibodies specific to an oxidized form of HD5 (ox-HD5), and established a method for immunologically detecting ox-HD5 by use of the same, and accomplished the following inventions.
(1) A method for immunologically detecting a human α-defensin HD5 with intramolecular disulfide bonds, the method comprising detecting the human α-defensin HD5 with the intramolecular disulfide bonds in a specimen by a sandwich method with use of a monoclonal antibody or an antigen binding fragment thereof according to the following a) and a monoclonal antibody or an antigen binding fragment thereof according to the following b):
   a) a monoclonal antibody or an antigen binding fragment thereof that specifically binds to the human α-defensin HD5 with the intramolecular disulfide bonds and comprises: a heavy chain variable region comprising the amino acid sequence of SEQ ID NO: 1 as CDR1, the amino acid sequence of SEQ ID NO: 2 as CDR2, and the amino acid sequence of SEQ ID NO: 3 as CDR3; and a light chain variable region comprising the amino acid sequence of SEQ ID NO: 4 as CDR1, the amino acid sequence of SEQ ID NO: 5 as CDR2, and the amino acid sequence of SEQ ID NO: 6 as CDR3; and
   b) a monoclonal antibody or an antigen binding fragment thereof that specifically binds to the human α-defensin HD5 with the intramolecular disulfide bonds and comprises: a heavy chain variable region comprising the amino acid sequence of SEQ ID NO: 7 as CDR1, the amino acid sequence of SEQ ID NO: 8 as CDR2, and the amino acid sequence of SEQ ID NO: 9 as CDR3; and a light chain variable region comprising the amino acid sequence of SEQ ID NO: 10 as CDR1, the amino acid sequence of SEQ ID NO: 11 as CDR2, and the amino acid sequence of SEQ ID NO: 12 as CDR3.
(2) The method according to (1), wherein
   the monoclonal antibody or the antigen binding fragment thereof according to a) comprises:
   a heavy chain variable region comprising the amino acid sequence of SEQ ID NO: 13 or an amino acid sequence having 90% or more sequence identity to the amino acid sequence of SEQ ID NO: 13; and
   a light chain variable region comprising the amino acid sequence of SEQ ID NO: 14 or an amino acid sequence having 90% or more sequence identity to the amino acid sequence of SEQ ID NO: 14, and
   the monoclonal antibody or the antigen binding fragment thereof according to b) comprises:
      a heavy chain variable region comprising the amino acid sequence of SEQ ID NO: 15 or an amino acid sequence having 90% or more sequence identity to the amino acid sequence of SEQ ID NO: 15; and
      a light chain variable region comprising the amino acid sequence of SEQ ID NO: 16 or an amino acid sequence having 90% or more sequence identity to the amino acid sequence of SEQ ID NO: 16.
(3) The method according to (1) or (2), wherein the monoclonal antibody or the antigen binding fragment thereof according to a) is used as a capture antibody, and the monoclonal antibody or the antigen binding fragment thereof according to b) is used as a detection antibody.
(4) The method according to any one of (1) to (3) comprising: a step of adding the specimen to a support on which the monoclonal antibody or the antigen binding fragment thereof according to a) is immobilized; a step of adding the labeled monoclonal antibody or the antigen binding fragment thereof according to b) to the support; and a step of detecting the monoclonal antibody or the antigen binding fragment thereof according to b) that binds to the human α-defensin HD5 with the intramolecular disulfide bonds.
(5) The method according to any one of (1) to (4), wherein the specimen is an extract with an aqueous medium from a freeze-dried and crushed human feces.
(6) A kit for immunologically detecting a human α-defensin HD5 with intramolecular disulfide bonds by a sandwich method, the kit comprising a monoclonal antibody or an antigen binding fragment thereof according to the following a) and a monoclonal antibody or an antigen binding fragment thereof according to the following b):
   a) a monoclonal antibody or an antigen binding fragment thereof that specifically binds to the human α-defensin HD5 with the intramolecular disulfide bonds and comprises: a heavy chain variable region comprising the amino acid sequence of SEQ ID NO: 1 as CDR1, the amino acid sequence of SEQ ID NO: 2 as CDR2, and the amino acid sequence of SEQ ID NO: 3 as CDR3; and a light chain variable region comprising the amino acid sequence of SEQ ID NO: 4 as CDR1, the amino acid sequence of SEQ ID NO: 5 as CDR2, and the amino acid sequence of SEQ ID NO: 6 as CDR3; and
   b) a monoclonal antibody or an antigen binding fragment thereof that specifically binds to the human α-defensin HD5 with the intramolecular disulfide bonds and comprises: a heavy chain variable region comprising the amino acid sequence of SEQ ID NO: 7 as CDR1, the amino acid sequence of SEQ ID NO: 8 as CDR2, and the amino acid sequence of SEQ ID NO: 9 as CDR3; and a light chain variable region comprising the amino acid sequence of SEQ ID NO: 10 as CDR1, the amino acid sequence of SEQ ID NO: 11 as CDR2, and the amino acid sequence of SEQ ID NO: 12 as CDR3.
(7) The kit according to (6), wherein the monoclonal antibody or the antigen binding fragment thereof according to a) is used as a capture antibody, and the monoclonal antibody or the antigen binding fragment thereof according to b) is used as a detection antibody.
(8) The kit according to (7), wherein the monoclonal antibody or the antigen binding fragment thereof according to a) is immobilized on a support, and the monoclonal antibody or the antigen binding fragment thereof according to b) is labeled.
(9) A monoclonal antibody or an antigen binding fragment thereof that specifically binds to a human α-defensin HD5 with intramolecular disulfide bonds and comprises: a heavy chain variable region comprising the amino acid sequence of SEQ ID NO: 1 as CDR1, the amino acid sequence of SEQ ID NO: 2 as CDR2, and the amino acid sequence of SEQ ID NO: 3 as CDR3; and a light chain variable region comprising the amino acid sequence of SEQ ID NO: 4 as CDR1, the amino acid sequence of SEQ ID NO: 5 as CDR2, and the amino acid sequence of SEQ ID NO: 6 as CDR3.
(10) A monoclonal antibody or an antigen binding fragment thereof that specifically binds to a human α-defensin HD5 with intramolecular disulfide bonds and comprises: a heavy chain variable region comprising the amino acid sequence of SEQ ID NO: 7 as CDR1, the amino acid sequence of SEQ ID NO: 8 as CDR2, and the amino acid sequence of SEQ ID NO: 9 as CDR3; and a light chain variable region comprising the amino acid sequence of SEQ ID NO: 10 as CDR1, the amino acid sequence of SEQ ID NO: 11 as CDR2, and the amino acid sequence of SEQ ID NO: 12 as CDR3.
(11) A nucleic acid encoding a monoclonal antibody or an antigen binding fragment thereof according to (9) or (10).
(12) A vector comprising the nucleic acid according to (11).
(13) A host cell comprising the nucleic acid according to (11).

### ADVANTAGEOUS EFFECTS OF INVENTION

A method and a kit for detecting an oxidized form of human α-defensin HD5, and antibodies used in the same, according to the present invention enables simple detection or measurement of ox-HD5 at a high sensitivity, and can be utilized in monitoring of intestinal environment, exploration of useful substances capable of controlling the intestinal environment, and investigation of new methods for preventing diseases relevant to the intestinal environment and strategies to cure such diseases.

### BRIEF DESCRIPTION OF DRAWINGS

FIG. 1 is the amino acid sequence of HD5 and positions of intramolecular disulfide bonds.
FIG. 2A is a mass spectrum of ox-HD5 by MALDI-TOF MS.
FIG. 2B is a mass spectrum of HD5 without any intramolecular disulfide bond (a reduced form of HD5, hereinafter represented as r-HD5), by MALDI-TOF MS.
FIG. 3 is a set of graphs representing sandwich ELISA standard curves for twenty-five combinations of five types of anti-ox-HD5 monoclonal antibodies as capture antibodies and the five types of anti-ox-HD5 monoclonal antibodies as detection antibodies.
FIG. 4 is a graph representing a standard curve of sandwich ELISA with the concentration of the detection antibody 39E-7 set to 0.5 mg/ml and the concentration of the capture antibody 12-1 set to 0.1, 0.5, 1, 5 or 10 mg/ml.
FIG. 5 is a graph representing absorbances in sandwich ELISA with the concentration of the detection antibody 39E-7 set to 0.5 mg/ml and the concentration of the capture antibody 12-1 set to 0.1, 0.5, 1, 5 or 10 mg/ml, at ox-HD5 concentration of 30 ng/ml.
FIG. 6 is a graph representing a standard curve of sandwich ELISA with the concentration of the capture antibody 12-1 set to 1 mg/ml and the concentration of the detection antibody 39E-7 set to 0.1, 0.5 or 1 mg/ml.
FIG. 7 is a graph representing absorbances in sandwich ELISA with the concentration of the capture antibody 12-1 set to 1 mg/ml and the concentration of the detection antibody 39E-7 set to 0.1, 0.5 or 1 mg/ml, at ox-HD5 concentration of 30 ng/ml.
FIG. 8 is a graph representing reactivities to r-HD5, r-HD6, reduced form (r-Crp1) and oxidized form (ox-Crp1) of cryptdin 1, and reduced form (r-Crp4) and oxidized form (ox-Crp4) of cryptdin 4 in sandwich ELISA with the concentration of the capture antibody 12-1 set to 1 mg/ml and the concentration of the detection antibody 39E-7 set to 0.5 mg/ml.
FIG. 9 is a set of scatter plots representing results of quantitative determination of ox-HD5 in human feces and serum by sandwich ELISA.

### DESCRIPTION OF EMBODIMENTS

A first aspect of the present invention relates to a method for immunologically detecting ox-HD5, comprising detecting ox-HD5 in a specimen by a sandwich method with use of a monoclonal antibody or an antigen binding fragment thereof according to the following a) and a monoclonal antibody or an antigen binding fragment thereof according to the following b):
a) a monoclonal antibody or an antigen binding fragment thereof that specifically binds to ox-HD5 and comprises: a heavy chain variable region comprising the amino acid sequence of SEQ ID NO: 1 as CDR1, the amino acid sequence of SEQ ID NO: 2 as CDR2, and the amino acid sequence of SEQ ID NO: 3 as CDR3; and a light chain variable region comprising the amino acid sequence of SEQ ID NO: 4 as CDR1, the amino acid sequence of SEQ ID NO: 5 as CDR2, and the amino acid sequence of SEQ ID NO: 6 as CDR3; and
b) a monoclonal antibody or an antigen binding fragment thereof that specifically binds to ox-HD5 and comprises: a heavy chain variable region comprising the amino acid sequence of SEQ ID NO: 7 as CDR1, the amino acid sequence of SEQ ID NO: 8 as CDR2, and the amino acid sequence of SEQ ID NO: 9 as CDR3; and a light chain variable region comprising the amino acid sequence of SEQ ID NO: 10 as CDR1, the amino acid sequence of SEQ ID NO: 11 as CDR2, and the amino acid sequence of SEQ ID NO: 12 as CDR3.

The present invention also provides as other aspects: a kit for immunologically detecting ox-HD5 with intramolecular disulfide bonds by the sandwich method, the kit comprising the monoclonal antibody or the antigen binding fragment thereof according to a) described above and the monoclonal antibody or the antigen binding fragment thereof according to b) described above; the monoclonal antibody or the antigen binding fragment thereof according to a) described above; and the monoclonal antibody or the antigen binding fragment thereof according to b) described above.

### Human α-defensin HD5

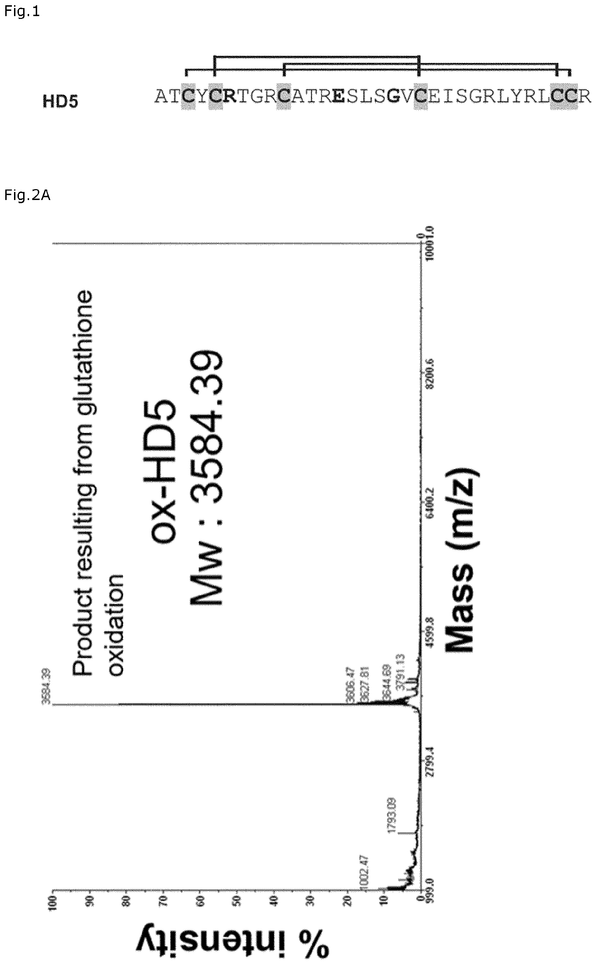

Human α-defensin has two isoforms, HD5 and HD6. HD5 (DEFA5, also referred to as defensin α5) is translated as a full-length prepeptide pre-HD5 (SEQ ID NO: 17) with 94 amino acid residues including a signal peptide at positions 1 to 19 in its N-terminus side. The signal peptide is cleaved out from the pre-HD5 to generate propeptide pro-HD5 (75 amino acids), and then 43 amino acid residues are cleaved out in the N-terminus side to generate a mature form of HD5 (32 amino acids, SEQ ID NO: 18). The amino acid sequence of the mature form of HD5 includes six cysteine residues that form three intramolecular disulfide bonds to generate ox-HD5. FIG. 1 illustrates the amino acid sequence of ox-HD5 and positions of the intramolecular disulfide bonds.

### Anti-ox-HD5 monoclonal antibody

Anti-ox-HD5 monoclonal antibodies according to the above a) and b) used in the present invention refers to an antibody specifically binding to ox-HD5, that is, HD5 with three intramolecular disulfide bonds. The term, specifically binding to ox-HD5, means binding preferentially to ox-HD5 over untargeted proteins such as a reduced form of HD5 without any intramolecular disulfide bond, for example, and does not mean never binding to untargeted proteins. The antibody specifically binding to ox-HD5 is also referred to as the antibody having high binding affinity to ox-HD5. The antibody can bind to ox-HD5 with at least twice, preferably at least 10 times, more preferably at least 20 times, most preferably at least 100 times higher affinity than the affinity with which the antibody binds to untargeted proteins. When an antibody can detect the presence of ox-HD5 without giving undesirable results such as false positive and false negative results typically, the antibody is considered to be the antibody specifically binding to ox-HD5.

In the present invention, the anti-ox-HD5 monoclonal antibody may belong to any class of immunoglobulin molecules (for example, IgG, IgE, IgM, IgD or IgA) and any subclass thereof, but is preferably IgG. The monoclonal antibody may originate from any one of species including mouse, rat, shark, rabbit, pig, hamster, camel, llama, goat and human, for example, and may be a chimeric antibody or a humanized antibody.

The heavy chain variable region of the anti-ox-HD5 monoclonal antibody according to a) has 90% or more, 91% or more, 92% or more, 93% or more, 94% or more, 95% or more, 96% or more, 97% or more, 98% or more, or 99% or more sequence identity to the amino acid sequence of SEQ ID NO: 13, and preferably has the same amino acid sequence as the amino acid sequence of SEQ ID NO: 13.

The light chain variable region of the anti-ox-HD5 monoclonal antibody according to a) has 90% or more, 91% or more, 92% or more, 93% or more, 94% or more, 95% or more, 96% or more, 97% or more, 98% or more, or 99% or more sequence identity to the amino acid sequence of SEQ ID NO: 14, and preferably has the same amino acid sequence as the amino acid sequence of SEQ ID NO: 14.

The heavy chain variable region of the anti-ox-HD5 monoclonal antibody according to b) has 90% or more, 91% or more, 92% or more, 93% or more, 94% or more, 95% or more, 96% or more, 97% or more, 98% or more, or 99% or more sequence identity to the amino acid sequence of SEQ ID NO: 15, and preferably has the same amino acid sequence as the amino acid sequence of SEQ ID NO: 15.

The light chain variable region of the anti-ox-HD5 monoclonal antibody according to b) has 90% or more, 91% or more, 92% or more, 93% or more, 94% or more, 95% or more, 96% or more, 97% or more, 98% or more, or 99% or more sequence identity to the amino acid sequence of SEQ ID NO: 16, and preferably has the same amino acid sequence as the amino acid sequence of SEQ ID NO: 16.

The identity of amino acid sequence is represented as a ratio of the number of identical amino acid residues to an alignment length, and the two compared amino acid sequences are aligned in such a way as to maximize the number of identical amino acid residues in accordance with conventional methods. The sequence identity can be determined by use of a sequence comparison program such as BLAST, for example, in accordance with any method well-known to a person skilled in the art.

The heavy chain variable regions and the light chain variable regions of the anti-ox-HD5 monoclonal antibodies according to a) and b) may contain deletion, substitution or addition of 10 or less, 9 or less, 8 or less, 7 or less, 6 or less, 5 or less, 4 or less, 3 or less, 2 or less, or one amino acid(s) in framework regions, namely amino acid sequences other than CDR. Here, the substitution of amino acid is preferably conservative substitution. The term "conservative substitution" refers to substituting a certain amino acid in the protein with another amino acid with similar properties (for example, charge, side chain size, hydrophobicity/hydrophilicity, backbone structure, rigidity and the like) without any influence on desired properties of the protein such as biological activity, affinity and/or specificity to its antigen. Examples of the conservative substitution include a substitution between amino acids such as glycine (Gly) - proline (Pro), glycine - alanine (Ala) or valine (Val), leucine (Leu) - isoleucine (Ile), glutamic acid (Glu) - glutamine (Gln), aspartic acid (Asp) - asparagine (Asn), cysteine (Cys) - threonine (Thr), threonine - serine (Ser) or alanine, and lysine (Lys) - arginine (Arg).

In the present invention, the antigen binding fragment is defined as a partial fragment of an antibody that retains the capability of specifically binding to the antigen. Examples of antigen binding fragment are not limited, but include Fab (fragment of antigen binding), Fab', F(ab')2, a single chain antibody (single chain Fv), a disulfide stabilized antibody (disulfide stabilized Fv) and a peptide containing CDR.

The anti-ox-HD5 monoclonal antibody and the antigen binding fragment thereof used in the present invention can be prepared by methods well-known to a person skilled in the art. For example, the anti-ox-HD5 monoclonal antibody can be prepared by immunizing a suitable animal with ox-HD5 as an antigen, then fusing B cells of the animal to myeloma cells to obtain a hybridoma, and then culturing the same. For example, see Meyaard et al. (1997) Immunity 7:283-290; Wright et al. (2000) Immunity 13:233-242; Kaithamana et al. (1999) J.Immunol. 163:5157-5164, and so on, for a hybridoma method.

ox-HD5 can be prepared by producing a polypeptide consisting of the amino acid sequence represented by SEQ ID NO: 18 in a chemical or genetic engineering way, and then using the polypeptide for a disulfide bond formation reaction used in a well-known protein refolding process. The disulfide bond formation reaction can be carried out typically by gently stirring in a weak alkaline aqueous solvent at approximately 4°C to 10°C for approximately 12 hours to 48 hours, or by gently stirring for oxidation at approximately 20°C to 30°C for approximately 12 hours to 48 hours in the presence of a redox agent (oxidized glutathione/reduced glutathione, cystine/cysteine, cysteamine/cystamine and the like) that is prepared by combining an oxidant with a reductant at an appropriate ratio.

ox-HD5 may be prepared by transforming a host cell with use of a nucleic acid encoding pre-HD5 or pro-HD5, and converting the pre-HD5 or pro-HD5 into ox-HD5 in the host cell. ox-HD5 may be utilized as it is as the antigen, or bound to various carrier proteins through a suitable condensing agent so as to be yielded as an immunogenic conjugate as the antigen. ox-HD5 may be used as the antigen with a suitable adjuvant.

The anti-ox-HD5 monoclonal antibody and the antigen binding fragment thereof used in the present invention can be prepared by genetic engineering methods that includes introducing expression vectors containing the nucleic acids encoding them into suitable host cells and expressing them in the host cells. The antibody preparation according to genetic engineering methods including preparation of nucleic acids encoding the anti-ox-HD5 monoclonal antibody and the antigen binding fragment thereof, types of host cells, methods for gene introduction, expression and purification of proteins and the like, can be carried out by methods well-known to the person skilled in the art according to instructions in experimental procedure manuals that describes in detail about various operations for genetical modification. The present invention also provides the nucleic acids encoding the anti-ox-HD5 monoclonal antibody and the antigen binding fragment thereof, the expression vector containing the nucleic acid, and the transformed host cell, as other aspects.

A preferred example of the anti-ox-HD5 monoclonal antibody according to a) is a monoclonal antibody (represented as monoclonal antibody 12-1 or 12-1 simply, hereinafter) produced by a hybridoma that has been internationally deposited with International Accession Number NITE BP-03086 (identification reference: 12-1) dated on Dec. 4, 2019 in #122, 2-5-8 Kazusakamatari, Kisarazu-shi, Chiba, Japan, Patent Microorganisms Depositary Center of the National Institute of Technology and Evaluation.

A preferred example of the anti-ox-HD5 monoclonal antibody according to b) is a monoclonal antibody (represented as monoclonal antibody 39E-7 or 39E-7 simply, hereinafter) produced by a hybridoma that has been internationally deposited with International Accession Number NITE BP-03087 (identification reference: 39E-7) dated on Dec. 4, 2019 in #122, 2-5-8 Kazusakamatari, Kisarazu-shi, Chiba, Japan, Patent Microorganisms Depositary Center of the National Institute of Technology and Evaluation.

A preferred example of the antigen binding fragment of the anti-ox-HD5 monoclonal antibody according to a) is an antigen binding fragment of the monoclonal antibody 12-1, and a preferred example of the antigen binding fragment of the anti-ox-HD5 monoclonal antibody according to b) is an antigen binding fragment of the monoclonal antibody 39E-7.

### Immunological detection method and kit for ox-HD5

Both of the anti-ox-HD5 monoclonal antibody and the antigen binding fragment thereof in the present invention are specifically bound to ox-HD5, and thereby can be used for immunological detection of ox-HD5 and utilized in affinity columns and the like for ox-HD5.

In addition, the anti-ox-HD5 monoclonal antibody or the antigen binding fragment thereof according to a) and the anti-ox-HD5 monoclonal antibody or the antigen binding fragment thereof according to b) recognize different epitopes of ox-HD5 and are simultaneously bound to one molecule of ox-HD5 to form a sandwich complex, thereby enabling the immunological detection of ox-HD5 in a specimen at a high sensitivity by the sandwich method with use of combination of these antibodies.

The immunological detection method for ox-HD5 includes all types of immunoassays utilizing antigen-antibody reactions, and examples thereof include ELISA, radio immunoassay, immunostaining of tissues or cells, immunoblotting, immunochromatography. In the present invention, it is preferred to employ the immunological detection by the sandwich method with use of the anti-ox-HD5 monoclonal antibody or the antigen binding fragment thereof according to a) and the anti-ox-HD5 monoclonal antibody or the antigen binding fragment thereof according to b). In particular, it is preferred to employ the immunological detection by the sandwich method with use of the anti-ox-HD5 monoclonal antibody or the antigen binding fragment thereof according to a) as a capture antibody and the anti-ox-HD5 monoclonal antibody or the antigen binding fragment thereof according to b) as a detection antibody, for example, sandwich ELISA.

The anti-ox-HD5 monoclonal antibody and the antigen binding fragment thereof according to the present invention can be immobilized on a suitable support or labeled with an appropriate labeling compound, for the purpose of the immunological detection of ox-HD5. In a preferred embodiment of the present invention, the monoclonal antibody or the antigen binding fragment thereof according to a) to be used as the capture antibody is immobilized on the support, while the monoclonal antibody or the antigen binding fragment thereof according to b) to be used as the detection antibody is labeled.

The support on which the antibody is to be immobilized, methods for immobilizing the antibody on the support, the labeling compound to be used in labeling the detection antibody, methods for labeling the antibody using the labeling compound, as well as operations for immunoassay using these, such as incubation conditions for the specimen and the support on which the antibody is immobilized, washing, blocking, and detection methods, for example, can be referred to methods well-known to the person skilled in the art, such as description of The Immunoassay Handbook: Theory and Applications of Ligand Binding, ELISA and Related Techniques (4TH), Elsevier, which is incorporated herein by reference in its entirety.

Examples of the labeling compound include fluorescent substances (e.g., FITC, rhodamine and the like), metal particles such as gold colloid, fluorescent microbeads such as Luminex (registered trademark, Luminex Corporation), pigment proteins (e.g., phycoerythrin, phycocyanin and the like), radioisotopes (e.g., ³H, ¹⁴C, ³²P, ³⁵S, ¹²⁵I, ¹³¹I and the like), enzymes (for example, peroxidase, alkaline phosphatase and the like), biotin and streptavidin.

The kit according to one aspect of the present invention comprises the monoclonal antibody or the antigen binding fragment thereof according to a) and the monoclonal antibody or the antigen binding fragment thereof according to b). The kit may further comprise a support such as a plate, and a reagent such as a blocking solution, a washing solution and a coloring substrate.

The specimen to be used in the immunological detection of ox-HD5 is preferably a biological sample derived from human, and examples thereof include tissues, cells, body fluids (for example, blood, lymph, saliva, mucus, bone marrow fluid, urine, semen, abdominal cavity fluid, and the like), feces and the like. The biological sample may be used as it is collected, or used after being subjected to pretreatment such as crushing, homogenization, centrifugal separation, concentration and dilution.

In the case of detecting ox-HD5 in feces, it is preferred to use an extract obtained by extracting a freeze-dried and crushed feces with an aqueous medium as the specimen. Such a specimen can be prepared typically by crushing a freeze-dried feces with use of a grinder such as bead grinder, then adding an aqueous medium generally used in the field of biochemistry without causing any negative influence on the biological sample, such as distilled water, physiological saline, phosphate buffer, phosphate buffered saline, for example, to the crushed feces, then shaking the resulting solution at approximately 4°C for 1 hour to 48 hours, preferably 4 hours to 36 hours, more preferably 8 hours to 24 hours, and then removing a solid content by centrifugal separation or filtration.

In the case of detecting ox-HD5 in blood, it is preferred to use serum or plasma as the specimen.

In a particularly preferred embodiment of the present invention, the immunological detection method for ox-HD5 comprises: a step of adding the specimen to the support on which the monoclonal antibody or the antigen binding fragment thereof according to a) is immobilized, the support being prepared by adding a solution containing the monoclonal antibody or the antigen binding fragment thereof according to a) at a concentration of 0.5 mg/ml or more, for example 0.5 to 10 mg/ml, preferably 1 to 10 mg/ml; a step of adding to the support the labeled monoclonal antibody or the antigen binding fragment thereof according to b) at a final concentration of 0.1 mg/ml or more, for example 0.1 to 1 mg/ml, preferably 0.5 to 1 mg/ml in a solution on the support during the addition; and a step of detecting the label of the monoclonal antibody or the antigen binding fragment thereof according to b) that is bound to human α-defensin HD5 with intramolecular disulfide bonds. The final concentration of the labeled monoclonal antibody or the antigen binding fragment thereof according to b) in the solution on the support during the addition can be adjusted by setting the concentration of a solution to be added in consideration of dilution with the residual solution resulting from the preceding. For example, in the case of removing the liquid from the support after the step of adding the specimen, the above final concentration is substantially equal to the concentration of the labeled monoclonal antibody or the antigen binding fragment thereof according to b) in the added solution, in view of almost no residual amount of the liquid on the support.

The present invention will be described more in detail with reference to the following Examples, but the present invention is not limited thereto.

### EXAMPLES

### Example 1. Preparation of anti-ox-HD5 monoclonal antibodies

### 1) Preparation of anti-ox-HD5

Chemical synthesis of mature HD5 (SEQ ID NO: 18) was outsourced to Thermo Fisher Scientific. The mature HD5 was dissolved in 0.1% acetic acid, then the pH was adjusted to 8.4 to 8.8 with 10% sodium hydroxide. The resulting solution was gently stirred at 4°C for 18 hours for an oxidation treatment. Subsequently, the solvent was substituted with 5% acetic acid with use of a dialysis membrane, and then the solution was removed by freeze-drying in vacuum. The resulting sample was dissolved in 0.1% trifluoroacetic acid, and then the solution thereof was subjected to a reverse-phase chromatography (C-18 column) to collect polypeptide fraction.

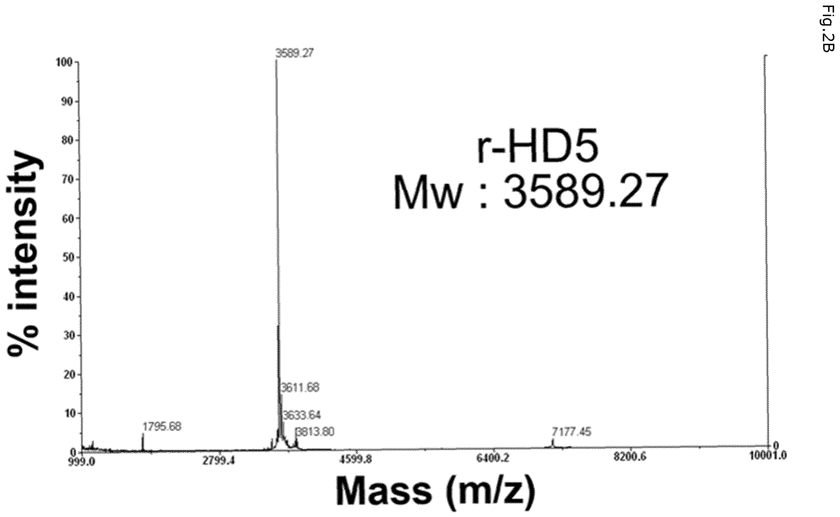

The polypeptide fraction and the synthesized polypeptide prior to the oxidation treatment were analyzed by MALDI-TOF MS (FIGS. 2A and 2B). As a result, the molecular weight of the oxidation-treated polypeptide fraction was confirmed to decrease by approximately 6 Da compared with that of r-HD5 (molecular weight: 3588.2). This indicates the formation of ox-HD5 with three intramolecular disulfide bonds resulting from the oxidation treatment.

Instead of the above oxidation treatment, another oxidization treatment was performed by dissolving mature HD5 in a glutathione oxidation solution (reduced glutathione (3 mM), oxidized glutathione (0.3 mM), and urea (8 M)), adjusting the pH to 8.4 with sodium bicarbonate (0.25 M), and then stirring the resulting solution gently for 18 hours at room temperature. This oxidation treatment also yielded ox-HD5 with three intramolecular disulfide bonds, which was confirmed by MALDI-TOF MS. ox-HD5 was used in Examples described below.

### 2) Preparation of hybridomas

Autoimmune disease mice (C3H/HeJJmsSlc-lpr/lpr available from Japan SLC, Inc.) injected with an adjuvant according to a conventional method were immunized with ox-HD5 by three times of intra-abdominal injection (0.2 mg each time). Later, cells were collected from the spleen of the immunized mice according to a conventional method, and then fused with a mouse myeloma cell line P3U1 with use of 50% polyethylene glycol 1500 (Sigma-Aldrich Co. LLC) to prepare hybridomas. Each hybridoma clone was screened by indirect ELISA, and subcloned four times by a limiting dilution method.

The indirect ELISA was carried out by the following procedures. Wells of a flat-bottomed microtiter plate (Nunc) were coated with 1 µg/ml of ox-HD5 dissolved in Ca²⁺/Mg²⁺⁻free phosphate buffered saline (PBS), and then incubated overnight at 4°C. After three times of washing with PBS containing 0.1% Tween 20 (PBS-T), culture supernatant from each hybridoma clone was added to the wells, and incubated at 37°C for 1 hour. After washing with PBS-T, horseradish peroxidase (HRP)-bound goat antimouse IgG (GE Healthcare Biosciences) was added, then the resulting solution was incubated for 45 minutes, and then washed. 100 µL of 3,3',5,5'-tetramethylbenzidine (TMB) substrate buffer (KPL) was added to each well, then the plate was incubated at 25°C for 30 minutes, and then absorbance at 405 nm was measured with use of a microplate reader Multiscan FC (Thermo Scientific).

The isotypes of monoclonal antibodies produced by five types of hybridoma clones (12-1, 16-1, 39E-7, 58-3, 60-10B) selected by the above procedures were determined with use of a mouse monoclonal antibody isotyping kit (F. Hoffmann-La Roche Ltd.). Results are listed in Table 1.

**[Table 1]**

| | | | | | |
|---|---|---|---|---|---|
| Clone No. | 12-1 | 16-1 | 39E-7 | 58-3 | 60.10B |
| Isotype | IgG₁ₖ | IgG₁ₖ | IgG₂ₐₖ | IgG₁ₖ | IgG₁ₖ |

### Example 2. Establishment of sandwich immunoassay

### 1) Selection of antibodies for immunoassay

Combination of antibodies suitable for immunoassay was selected by performing a sandwich ELISA with use of the five types of the monoclonal antibodies either as a capture antibody or a detection antibody by the following procedures.

Each of the monoclonal antibodies obtained in Example 1 was dissolved in a 50 mM sodium carbonate-sodium bicarbonate buffer (pH 9.6) to obtain 10 mg/ml solution. 100 µl of this solution was added to each well of the microtiter plate, and then incubated overnight at 4°C. After the plate was washed three times with PBS-T, 200 µl of 25% Block Ace (DS Pharma Biomedical) was added, and blocking was performed at 25°C for 1 hour. The plate washed with PBS-T was used as a plate on which the capture antibody was immobilized, in the immunoassay. The detection antibody was prepared by labeling each monoclonal antibody with use of biotin (Dojindo Laboratories) according to a manufacturer's protocol.

As a standard, 100 µl of 10 or 30 ng/ml solution of ox-HD5 dissolved in PBS-T was added to each well of the plate on which the capture antibody was immobilized, and then incubated at 25°C for 2 hours. After three times of washing with PBS (PBS-T), 100 µl of 0.5 mg/ml solution of the detection antibody was added to the wells and then incubated at 25°C for 1 hour. Next, 100 µl of Streptavidin-HRP conjugate (GE Healthcare Biosciences, 1:5000 dilution) was added to the wells, and then incubated at 25°C for 1 hour. After washing, 100 µl of TMB substrate buffer was added to the wells, then incubated at 25°C for 30 minutes, and then 100 µl of 0.6 N H₂SO₄ was added to quench the reaction. The absorbances at 450 nm (absorption wavelength) and 620 nm (reference wavelength) were measured with use of the microplate reader, and then the difference between both absorbances was calculated as a measurement value. The measurement value was represented as an average value ± standard deviation.

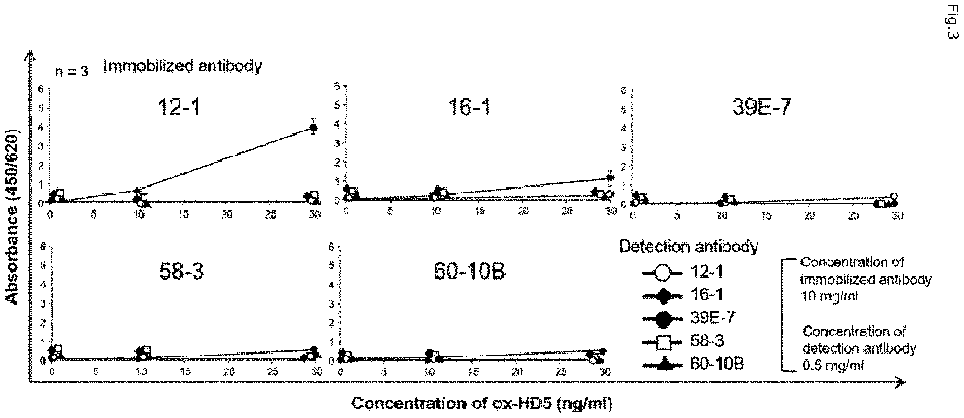

Results are illustrated in FIG. 3. The highest detection sensitivity was confirmed when the capture antibody was 12-1 and the detection antibody was 39E-7.

### 2) Optimization

For the combination of the capture antibody 12-1 with the detection antibody 39E-7, optimum concentrations of the antibodies were investigated. First, sandwich ELISA was performed in a similar manner to the above 1) with the detection antibody 39E-7 at a concentration of 0.5 mg/ml and the capture antibody 12-1 at a concentration of 0.1, 0.5, 1, 5, or 10 mg/ml, and a standard curve for ox-HD5 at concentrations from 0 to 30 ng/ml was prepared (n = 3). The statistical analysis was carried out by Student's t-test, and the P value < 0.05 was considered statistically significant.

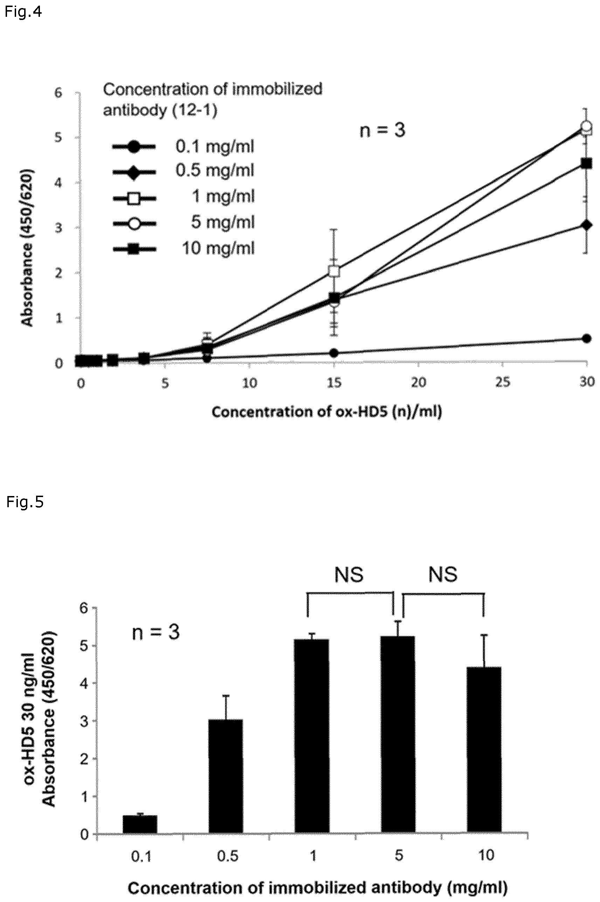

Results are illustrated in FIGS. 4 and 5. The highest absorbance of the 1 mg/ml of the concentration of the capture antibody was confirmed when the concentration of ox-HD5 was 15 ng/ml. When the concentration of ox-HD5 was 30 ng/ml, the absorbance was increased depending on the concentration of the capture antibody, and no significant difference was recognized among 1, 5 and 10 mg/ml. In view of the foregoing, the optimum concentration of the capture antibody was determined to be 1 mg/ml.

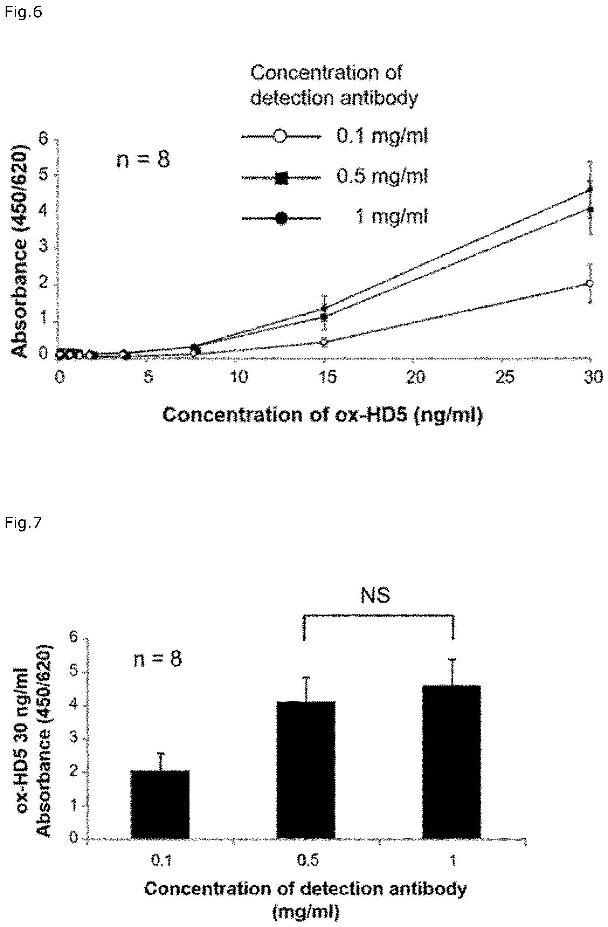

Next, sandwich ELISA was performed in a similar manner with the capture antibody 12-1 at a concentration of 1 mg/ml and the detection antibody 39E-7 at a concentration of 0.1, 0.5, or 1 mg/ml, and a standard curve was prepared (n = 8). Results are illustrated in FIGS. 6 and 7. When ox-HD5 concentration was 15 or 30 ng/ml, no significant difference was recognized between the absorbances of 0.5 and 1 mg/ml of the detection antibody. In view of the foregoing, the optimum concentration of the detection antibody was determined to be 0.5 mg/ml.

### 3) Confirmation of specificity

In order to investigate specificity of assay systems constructed in 1) and 2) mentioned above, the reactivity to r-HD5, a reduced form (r-HD6) of HD6 (SEQ ID NO: 19) that is an isoform of HD5, and oxidized and reduced forms (ox-Crp1, r-Crp1, ox-Crp4 and r-Crp4) of two types of mouse α-defensin (Crp1 and Crp4), was studied. Chemical synthesis of all of these peptides was outsourced to Thermo Fisher Scientific. The oxidized forms of Crp1 and Crp4 were prepared according to a previous report (K. Nakamura et al., Analytical biochemistry. 2013, 443(2), 124-131). Sandwich ELISA was performed in a similar manner with the capture antibody 12-1 at a concentration of 1 mg/ml and the detection antibody 39E-7 at a concentration of 0.5 mg/ml, and a standard curve for each antigen at concentrations from 0 to 60 ng/ml was prepared.

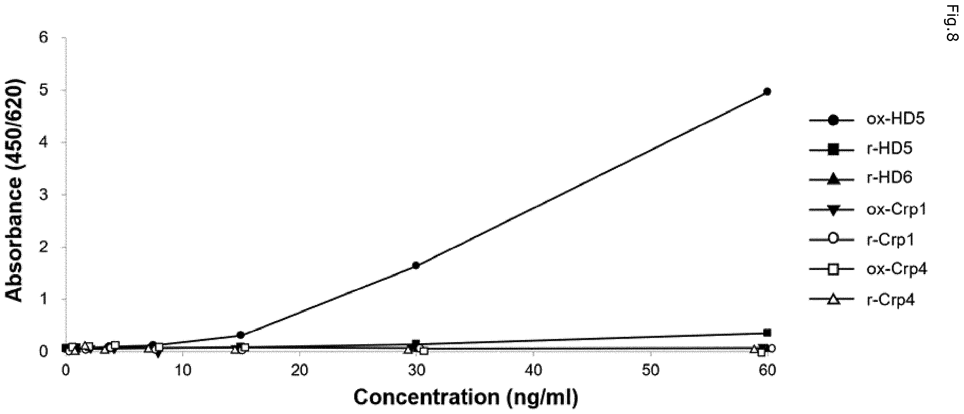

Results are illustrated in FIG. 8. In the case of using r-HD5 as the antigen, a slight increase in the absorbance was observed at a concentration of 60 ng/ml. Meanwhile, in the case of using r-HD6, ox-Crp1, r-Crp1, ox-Crp4 and r-Crp4 as the antigen, the absorbance was not increased. Thus, the present assay system was confirmed to have high affinity and specificity to ox-HD5.

### Example 3. CDR analysis for monoclonal antibodies

CDR sequences of the monoclonal antibodies 12-1 and 39E-7 were analyzed with use of SMART cDNA library preparation kit (Takara Bio Inc.). Specifically, total RNA was extracted from the hybridoma clones 12-1 and 39E-7. Then, cDNA was prepared with use of the extracted RNA as a template as well as 3'-terminal side IgG1 heavy chain specific primer, IgG2a heavy chain specific primer or κ light chain specific primer. PCR amplification was carried out with use of the cDNA as a template as well as 5'-terminal side specific primer included in the kit as a forward primer, 3'-terminal side IgG1 heavy chain specific primer, IgG2a heavy chain specific primer or κ light chain specific primer as reverse primers. The nucleic acid sequences of the amplified DNA fragments were analyzed with use of a DNA sequencer, and then the obtained sequence information was searched on igBLAST database (http://www.ncbi.nlm.nih.gov/igblast/). As a result, both of the monoclonal antibodies 12-1 and 39E-7 were confirmed to have new CDR sequence combinations. Table 2 lists the amino acid sequences of the heavy chains CDR1 to 3 and the variable region for each antibody. Table 3 lists the amino acid sequences of the light chains CDR1 to 3 and the variable region.

**[Table 2]**

| Heavy chain | Primary VH sequence | CDR1 | CDR2 | CDR3 |
|---|---|---|---|---|
| 12-1 | QVQLQQSGAELARPGASVKLSCKASGYT FTSYGISWVKQRTGQGLEWIGEIYPRSGN TYYNEKFKGKATLTADKSSSTAYMQLSSL TSEDSAVYFCARDYDGFPYFDYWGQGTT LTVSS (SEQ ID NO: 13) | SYGIS (SEQ ID NO: 1) | EIYPRSGNTYYNEKFKG (SEQ ID NO: 2) | DYDGFPYFDY (SEQ ID NO: 3) |
| 39E-7 | EVQLQQSGPELVKPGASVKISCKASGYSF TGSYMNWVKQSPEKSLEWIGEINPSTGG TTHNQKFKAKATLTVDTSSSTFYMQLKS LTSEDSAVYYCARDYAGSYDWYFDVWG AGTTVTVSS (SEQ ID NO: 15) | GSYMN (SEQ ID NO: 7) | EINPSTGGTTHNQKFKA (SEQ ID NO: 8) | DYAGSYDWYFDV (SEQ ID NO: 9) |

**[Table 3]**

| Light chain | Primary VL sequence | CDR1 | CDR2 | CDR3 |
|---|---|---|---|---|
| 12-1 | DIVLTQSPASLAVSLGQRATISCRASESVDS YGYSFMHWYQQKPGQPPKLLIYRASNLES GIPARFSGSGSRTDFTLTINPVEADDVATY YCQQSNEDPRTFGGGTKLEIK (SEQ ID NO: 14) | RASESVDSYGYSFMH (SEQ ID NO: 4) | RASNLES (SEQ ID NO: 5) | QQSNEDPRT (SEQ ID NO: 6) |
| 39E-7 | DIKMTQSPSSMYASLGERVTITCKASQDIN SYLSWFQQKPGKSPKTLIYRANRLVDGVP SRFSGSGSGQDYSLTISSLEYEDMGIYYCL QYDEFPYTFGGGTKLEII (SEQ ID NO: 16) | KASQDINSYLS (SEQ ID NO: 10) | RANRLVD (SEQ ID NO: 11) | LQYDEFPYT (SEQ ID NO: 12) |

The hybridoma clones 12-1 and 39E-7 were internationally deposited with Accession Numbers NITE BP-03086 (identification reference: 12-1) and NITE BP-03087 (identification reference: 39E-7) in Patent Microorganisms Depositary Center of the National Institute of Technology and Evaluation.

### Example 4 Quantitative determination of ox-HD5 in human specimen by sandwich ELISA

Serum and feces were collected from healthy volunteers (30 to 80 years old, total five volunteers). The feces were freeze-dried for 16 hours, and then crushed using a bead beater type homogenizer (Beads Crusher µT-12) with stainless beads. 500 µL of PBS (-) was added per 50 mg of fecal powders, and shaken for 16 hours and subjected to centrifugal separation (20,400 G, 10 minutes, 4°C) to obtain the supernatant as the measurement sample. Sandwich ELISA was carried out under the optimum condition determined in Example 2.

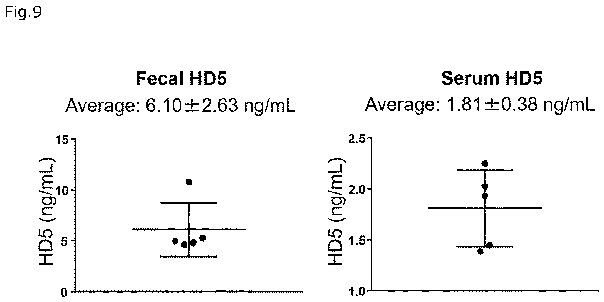

Results are illustrated in FIG. 9. An average of 6.10 ± 2.63 ng/mL of ox-HD5 was detected in the fecal extracts and 1.81 ± 0.38 ng/mL in the serum. It was confirmed that ox-HD5 could be quantified in both fecal extracts and serum.

### [Sequence Listing Free Text]

SEQ ID NO: 1: Amino acid sequence of heavy chain CDR1 of monoclonal antibody 12-1
SEQ ID NO: 2: Amino acid sequence of heavy chain CDR2 of monoclonal antibody 12-1
SEQ ID NO: 3: Amino acid sequence of heavy chain CDR3 of monoclonal antibody 12-1
SEQ ID NO: 4: Amino acid sequence of light chain CDR1 of monoclonal antibody 12-1
SEQ ID NO: 5: Amino acid sequence of light chain CDR2 of monoclonal antibody 12-1
SEQ ID NO: 6: Amino acid sequence of light chain CDR3 of monoclonal antibody 12-1
SEQ ID NO: 7: Amino acid sequence of heavy chain CDR1 of monoclonal antibody 39E-7
SEQ ID NO: 8: Amino acid sequence of heavy chain CDR2 of monoclonal antibody 39E-7
SEQ ID NO: 9: Amino acid sequence of heavy chain CDR3 of monoclonal antibody 39E-7
SEQ ID NO: 10: Amino acid sequence of light chain CDR1 of monoclonal antibody 39E-7
SEQ ID NO: 11: Amino acid sequence of light chain CDR2 of monoclonal antibody 39E-7
SEQ ID NO: 12: Amino acid sequence of light chain CDR3 of monoclonal antibody 39E-7
SEQ ID NO: 13: Amino acid sequence of heavy chain of monoclonal antibody 12-1
SEQ ID NO: 14: Amino acid sequence of light chain of monoclonal antibody 12-1
SEQ ID NO: 15: Amino acid sequence of heavy chain of monoclonal antibody 39E-7
SEQ ID NO: 16: Amino acid sequence of light chain of monoclonal antibody 39E-7
SEQ ID NO: 17: Amino acid sequence of pre-HD5
SEQ ID NO: 18: Amino acid sequence of mature HD5
SEQ ID NO: 19: Amino acid sequence of mature HD6

## Claims

1. A method for immunologically detecting a human α-defensin HD5 with intramolecular disulfide bonds, the method comprising detecting the human α-defensin HD5 with the intramolecular disulfide bonds in a specimen by a sandwich method with use of a monoclonal antibody or an antigen binding fragment of the monoclonal antibody according to the following a) and a monoclonal antibody or an antigen binding fragment of the monoclonal antibody according to the following b):
a) a monoclonal antibody or an antigen binding fragment of the monoclonal antibody that specifically binds to the human α-defensin HD5 with the intramolecular disulfide bonds and comprises: a heavy chain variable region comprising the amino acid sequence of SEQ ID NO: 1 as CDR1, the amino acid sequence of SEQ ID NO: 2 as CDR2, and the amino acid sequence of SEQ ID NO: 3 as CDR3; and a light chain variable region comprising the amino acid sequence of SEQ ID NO: 4 as CDR1, the amino acid sequence of SEQ ID NO: 5 as CDR2, and the amino acid sequence of SEQ ID NO: 6 as CDR3; and
b) a monoclonal antibody or an antigen binding fragment of the monoclonal antibody that specifically binds to the human α-defensin HD5 with the intramolecular disulfide bonds and comprises: a heavy chain variable region comprising the amino acid sequence of SEQ ID NO: 7 as CDR1, the amino acid sequence of SEQ ID NO: 8 as CDR2, and the amino acid sequence of SEQ ID NO: 9 as CDR3; and a light chain variable region comprising the amino acid sequence of SEQ ID NO: 10 as CDR1, the amino acid sequence of SEQ ID NO: 11 as CDR2, and the amino acid sequence of SEQ ID NO: 12 as CDR3.

2. The method according to claim 1, wherein
the monoclonal antibody or the antigen binding fragment of the monoclonal antibody according to a) comprises:
a heavy chain variable region comprising the amino acid sequence of SEQ ID NO: 13 or an amino acid sequence having 90% or more sequence identity to the amino acid sequence of SEQ ID NO: 13; and
a light chain variable region comprising the amino acid sequence of SEQ ID NO: 14 or an amino acid sequence having 90% or more sequence identity to the amino acid sequence of SEQ ID NO: 14, and wherein
the monoclonal antibody or the antigen binding fragment of the monoclonal antibody according to b) comprises:
a heavy chain variable region comprising the amino acid sequence of SEQ ID NO: 15 or an amino acid sequence having 90% or more sequence identity to the amino acid sequence of SEQ ID NO: 15; and
a light chain variable region comprising the amino acid sequence of SEQ ID NO: 16 or an amino acid sequence having 90% or more sequence identity to the amino acid sequence of SEQ ID NO: 16.

3. The method according to claim 1 or 2, wherein the monoclonal antibody or the antigen binding fragment of the monoclonal antibody according to a) is used as a capture antibody, and the monoclonal antibody or the antigen binding fragment of the monoclonal antibody according to b) is used as a detection antibody.

4. The method according to any one of claims 1 to 3, comprising: a step of adding the specimen to a support on which the monoclonal antibody or the antigen binding fragment of the monoclonal antibody according to a) is immobilized; a step of adding the labeled monoclonal antibody or the antigen binding fragment of the monoclonal antibody according to b) to the support; and a step of detecting the monoclonal antibody or the antigen binding fragment of the monoclonal antibody according to b) that binds to the human α-defensin HD5 with the intramolecular disulfide bonds.

5. The method according to any one of claims 1 to 4, wherein the specimen is an extract with an aqueous medium from a freeze-dried and crushed human feces.

6. A kit for immunologically detecting a human α-defensin HD5 with intramolecular disulfide bonds by a sandwich method, the kit comprising a monoclonal antibody or an antigen binding fragment of the monoclonal antibody according to the following a) and a monoclonal antibody or an antigen binding fragment of the monoclonal antibody according to the following b):
a) a monoclonal antibody or an antigen binding fragment of the monoclonal antibody that specifically binds to the human α-defensin HD5 with the intramolecular disulfide bonds and comprises: a heavy chain variable region comprising the amino acid sequence of SEQ ID NO: 1 as CDR1, the amino acid sequence of SEQ ID NO: 2 as CDR2, and the amino acid sequence of SEQ ID NO: 3 as CDR3; and a light chain variable region comprising the amino acid sequence of SEQ ID NO: 4 as CDR1, the amino acid sequence of SEQ ID NO: 5 as CDR2, and the amino acid sequence of SEQ ID NO: 6 as CDR3; and
b) a monoclonal antibody or an antigen binding fragment of the monoclonal antibody that specifically binds to the human α-defensin HD5 with the intramolecular disulfide bonds and comprises: a heavy chain variable region comprising the amino acid sequence of SEQ ID NO: 7 as CDR1, the amino acid sequence of SEQ ID NO: 8 as CDR2, and the amino acid sequence of SEQ ID NO: 9 as CDR3; and a light chain variable region comprising the amino acid sequence of SEQ ID NO: 10 as CDR1, the amino acid sequence of SEQ ID NO: 11 as CDR2, and the amino acid sequence of SEQ ID NO: 12 as CDR3.

7. The kit according to claim 6, wherein the monoclonal antibody or the antigen binding fragment of the monoclonal antibody according to a) is used as a capture antibody, and the monoclonal antibody or the antigen binding fragment of the monoclonal antibody according to b) is used as a detection antibody.

8. The kit according to claim 7, wherein the monoclonal antibody or the antigen binding fragment of the monoclonal antibody according to a) is immobilized on a support, and the monoclonal antibody or the antigen binding fragment of the monoclonal antibody according to b) is labeled.

9. A monoclonal antibody or an antigen binding fragment of the monoclonal antibody that specifically binds to a human α-defensin HD5 with intramolecular disulfide bonds and comprises: a heavy chain variable region comprising the amino acid sequence of SEQ ID NO: 1 as CDR1, the amino acid sequence of SEQ ID NO: 2 as CDR2, and the amino acid sequence of SEQ ID NO: 3 as CDR3; and a light chain variable region comprising the amino acid sequence of SEQ ID NO: 4 as CDR1, the amino acid sequence of SEQ ID NO: 5 as CDR2, and the amino acid sequence of SEQ ID NO: 6 as CDR3.

10. A monoclonal antibody or an antigen binding fragment of the monoclonal antibody that specifically binds to a human α-defensin HD5 with intramolecular disulfide bonds and comprises: a heavy chain variable region comprising the amino acid sequence of SEQ ID NO: 7 as CDR1, the amino acid sequence of SEQ ID NO: 8 as CDR2, and the amino acid sequence of SEQ ID NO: 9 as CDR3; and a light chain variable region comprising the amino acid sequence of SEQ ID NO: 10 as CDR1, the amino acid sequence of SEQ ID NO: 11 as CDR2, and the amino acid sequence of SEQ ID NO: 12 as CDR3.

11. A nucleic acid encoding the monoclonal antibody or the antigen binding fragment of the monoclonal antibody according to claim 9 or 10.

12. A vector comprising the nucleic acid according to claim 11.

13. A host cell comprising the nucleic acid according to claim 11.
